# EUROPÄISCHE PATENTANMELDUNG

(11) **EP 1 839 576 A1**
(43) Veröffentlichungstag der Anmeldung: **03.10.2007**
(21) Anmeldenummer: 06006523.2
(22) Anmeldetag: 29.03.2006
(51) Int. Cl.: A61B 5/15, A61B 5/00

(54) **Testsystem mit Testeinheit zum Untersuchen von Körperflüssigkeiten**

(71) Anmelder: F.HOFFMANN-LA ROCHE AG, 4070 Basel (CH); Roche Diagnostics GmbH, 68305 Mannheim (DE)
(72) Erfinder: Korner, Stephan, 6330 Cham (CH); Niederberger, Brigitte, 8800 Thalwil (CH); Schnarrwyler, Denise, 6006 Luzern (CH)
(74) Vertreter: Pfiz, Thomas

(57) **Zusammenfassung**

Die Erfindung betrifft eine Testeinheit zum Untersuchen von Körperflüssigkeiten mit einem Sammelelement (14), das einen mikrofluidischen Sammelbereich (36) für die Körperflüssigkeit aufweist, und einem Testelement (16), das durch Auslenkung eines Transferteils (34) des Sammelelements (14) mit dem Sammelbereich (36) zur Übertragung von Körperflüssigkeit in fluidische Verbindung bringbar ist. Erfindungsgemäß wird vorgeschlagen, dass der Transferteil (34) gegen einen Niederhalter (20) vorgespannt ist, wobei durch Lösen des Niederhalters (20) die Auslenkung des Transferteils (34) zu dem Testelement (16) hin auslösbar ist.

## Beschreibung

Die Erfindung betrifft eine Testeinheit und ein Testsystem zum Untersuchen von Körperflüssigkeiten wie Blut gemäß dem Oberbegriff der Patentansprüche 1 bzw. 16.

Aus der WO 2005/084530 ist eine Sammelvorrichtung für Körperflüssigkeiten bekannt, bei der ein bewegliches Teil eines Fließpfads für die Körperflüssigkeit durch ein Kontaktierungsmittel aktiv mit einem Empfangsmittel für die Flüssigkeit in Verbindung gebracht wird, indem beispielsweise durch einen mechanischen oder magnetischen Aktuator die Bewegung angetrieben wird. Damit kann eine kleine Flüssigkeitsmenge zu einem definierten Zeitpunkt einer Verarbeitung in einem Testprozess unterzogen werden. Im Hinblick auf die diesbezüglichen Voraussetzungen und Vorteile wird explizit auf die genannte Druckschrift Bezug genommen und deren Inhalt hier aufgenommen. Die aktive Kontaktierung erfordert jedoch eine geräteseitige Antriebseinheit, wodurch der Herstellungsaufwand erhöht und der Prozessablauf komplizierter wird. Insbesondere muss auch für die erforderliche Genauigkeit im Zusammenwirken zwischen dem Geräteantrieb und dem bewegten Element als Teil eines Disposables gesorgt werden.

Ausgehend hiervon liegt der Erfindung die Aufgabe zugrunde, die im Stand der Technik bekannten Einheiten bzw. Systeme weiter zu entwickeln, so dass mit einfacheren Mitteln ein gesteuerter Messablauf mit hoher Zuverlässigkeit ermöglicht wird.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, durch eine inhärente Transferbewegung auf einen externen Antrieb verzichten zu können. Dementsprechend wird erfindungsgemäß vorgeschlagen, dass der Transferteil gegen einen Niederhalter vorgespannt ist, wobei durch Lösen des Niederhalters die Auslenkung des Transferteils zu dem Testelement hin auslösbar ist. Durch die Vorspannung kann eine selbsttätige Bewegung zwischen einem Ausgangszustand und einem Kontaktzustand stattfinden, wobei der Ausgangszustand durch den Niederhalter als Rückhaltemittel erhalten wird, bis die Auslösung erfolgt. Hierfür stellen sich nur geringe Genauigkeitsanforderungen, da die Bewegung des Transferteils als solche keiner externen Steuerung bzw. Führung bedarf. Im Übrigen werden aber die bereits erwähnten Vorteile einer zeitlich definierten Triggerung des Testprozesses voll erreicht. Dies wirkt sich vor allem positiv bei Einmalartikeln aus, bei denen durch mikrofluidische Prozesse vorzugsweise unter Kapillarwirkung kleinste Probenmengen (beispielsweise Mikroliter und weniger) vom Ort der Gewinnung zum Ort der Testausführung übertragen werden.

Vorteilhafterweise verläuft der Sammelbereich zumindest teilweise über das Transferteil, wobei sich der Sammelbereich und das Testelement vor der Auslenkung körperlich getrennt im Abstand voneinander und nach der Auslenkung in Kontakt miteinander befinden. Auf diese Weise kann die Flüssigkeitsaufnahme auch unter schwierigen Bedingungen erfolgen, während erst nach erfolgreichem Abschluss des Sammelvorgangs eine gezielte Probenübertragung stattfindet.

Eine baulich vorteilhafte Ausführung sieht vor, dass der Transferteil mit einer Vorbiegung vorzugsweise als Biegearm an dem Sammelelement angebracht ist.

Für eine inhärente Transportbewegung durch Verformung ist es von Vorteil, wenn der Transferteil durch Einwirkung innerer Spannungen und/oder Oberflächenspannungen unter Biegeverformung auslenkbar ist. Dies lässt sich dadurch realisieren, dass der Transferteil durch eine unterschiedliche Oberflächenbehandlung an verschiedenen Seiten eine die Auslenkung bewirkende Biegespannung aufweist. Eine weitere Möglichkeit besteht darin, dass der Transferteil durch unterschiedliche Materiallagen nach Art eines Bimetallstreifens auslenkbar ist. Herstellungstechnisch besonders einfach ist es, wenn der Transferteil durch einen Ätzprozess aus einem flachen Substrat gebildet ist, wobei durch unterschiedliche Ätztiefen an verschiedenen Seiten des Transferteils resultierende innere Biegespannungen in dem Transferteil bestehen. Auf diese Weise ist kein zusätzlicher Biegeprozess erforderlich, da die Biegevorspannung bereits durch einen Prozessschritt eines für die Massenfertigung geeigneten formgebenden Verfahrens erhalten wird.

Eine weitere vorteilhafte Ausführung sieht vor, dass das Sammelelement ein Stechorgan zum Einstechen in ein Körperteil, beispielsweise in den Finger aufweist, wobei der Sammelbereich über den Einstich mit Körperflüssigkeit beaufschlagbar ist.

Um den Ausgangszustand des Transferteils auf einfache Weise festzulegen, kann der Niederhalter durch eine trennbare Materialbrücke zwischen dem Sammelelement und dem Transferteil gebildet ist, wobei die Auslenkung des Transferteils unter Durchtrennen der Materialbrücke auslösbar ist. Dies lässt sich besonders einfach dadurch bewerkstelligen, dass das Sammelelement und der Niederhalter aus einem Flachmaterial, insbesondere einem Edelstahlblech einstückig geformt sind, wobei der Niederhalter durch mindestens einen trennbaren Steg gebildet ist.

Alternativ ist es auch ohne besondere Herstellungsschwierigkeiten möglich, dass der Niederhalter durch einen auf das Sammelelement aufgebrachten und dabei einen Abschnitt des Transferteils übergreifenden Klebestreifen oder ein Laminat gebildet ist.

Um eine vorzeitige Flüssigkeitsübertragung zu verhindern, ist es von Vorteil, wenn das Testelement im Ausgangszustand vorzugsweise durch einen Abstandshalter auf dem Sammelelement in einem vorgegebenen Abstand zu dem Transferteil angeordnet ist.

Vorteilhafterweise bilden das Sammelelement und der Transferteil eine als disposibles Einmalprodukt bestimmte Baueinheit.

Um eine gezielte Übergabe auch kleinster Probenmengen zu ermöglichen ist es vorteilhaft, wenn der Transferteil im Kontaktzustand unter Einhaltung eines vorgegebenen Kontaktwinkels mit dem Testelement in Eingriff kommt.

Das Testelement kann ein über eine Öffnung des Sammelbereichs - speziell eine längsseitige Öffnung eines Kapillarkanals - mit der Körperflüssigkeit beaufschlagbares Reagenzfeld aufweisen.

Die Erfindung umfasst auch Testsystem zum Untersuchen von Körperflüssigkeiten wie Blut, bei welchem der Transferteil gegen einen Niederhalter an dem Sammelelement vorgespannt ist, wobei durch Lösen des Niederhalters mittels eines geräteseitigen Auslösers die Auslenkung auslösbar ist.

Hierbei ist es möglich, dass der Auslöser durch eine berührungslos arbeitende Trenneinheit, insbesondere einen Laser gebildet ist. Eine andere Variante sieht vor, dass der Auslöser ein mechanisch wirksames Schneidmittel aufweist. Denkbar ist es auch, dass der Auslöser durch einen thermischen Aktuator zur Triggerung einer Biegeverformung unter Wärmeeinwirkung gebildet ist.

Vorteilhafterweise wird das Testelement im System unabhängig von dem Sammelelement gehandhabt bzw. positioniert, insbesondere um eine Flüssigkeitsmenge auf eine gesonderte Messstelle zu übertragen.

Im Folgenden wird die Erfindung anhand der in der Zeichnung schematisch dargestellten Ausführungsbeispiele näher erläutert. Es zeigen:
- Fig. 1: ein Testsystem mit Sammel- und Testelement zum Aufnehmen und Untersuchen von Blut in einem Blockdiagramm;
- Fig. 2: das Sammelelement mit einem Transferteil für die Blutprobe in einem vorgebogenen Zustand in perspektivischer Ansicht;
- Fig. 3: das Sammelelement nach Fig. 2 in einer vereinfachten Seitenansicht und Draufsicht;
- Fig. 4: das Sammelelement in einer Fig. 3 entsprechenden Darstellung zusätzlich mit Niederhalter und Testelement im Ausgangszustand;
- Fig. 5: das Sammelelement entsprechend Fig. 4 in ausgelöstem Zustand; und
- Fig. 6 und 7: eine weitere Ausführungsform eines Sammelelements mit einem Trennsteg als Niederhalter in Fig. 4 und 5 entsprechenden Darstellungen.

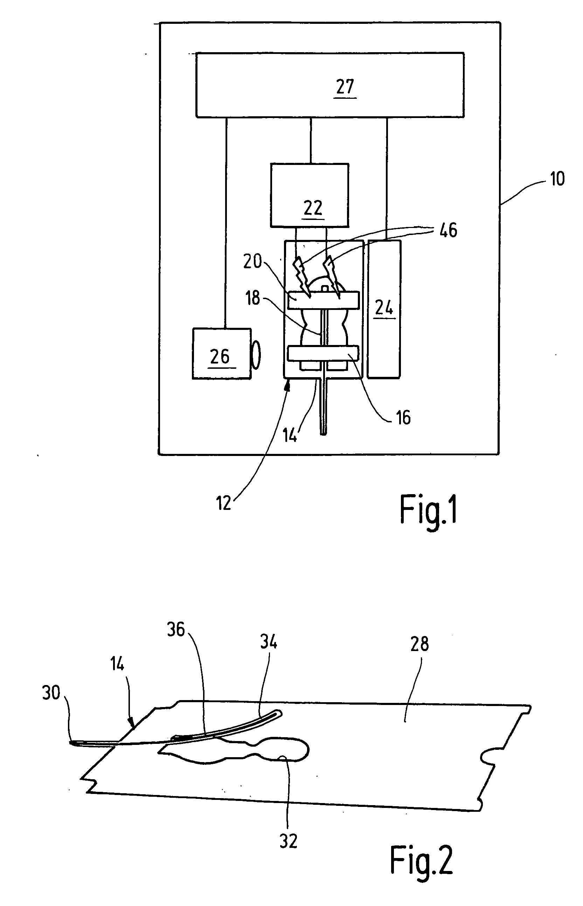

Das in Fig. 1 gezeigte Testsystem 10 ist bevorzugt als tragbares Blutzuckermessgerät ausgebildet und ermöglicht den Einsatz von disposiblen Testeinheiten 12 für die Selbstbestimmung von Blutzucker durch den Patienten. Zu diesem Zweck umfasst jede Testeinheit 12 ein Sammelelement 14 zur Aufnahme einer mikroskopischen Blutmenge und ein Testelement 16, das durch Auslenkung eines Transferteils 18 des Sammelelements 14 mit Blut beaufschlagbar ist, wobei der Transferteil 18 durch einen Niederhalter 20 im Ausgangszustand von dem Testelement 16 entfernt gehalten ist.

Als weitere Systemeinheiten sind ein Auslöser 22 für den Niederhalter 20, ein Stechantrieb 24 für das Sammelelement 14 und eine Messeinheit 26 für eine vorzugsweise optische Abtastung des Testelements 16 vorgesehen. Eine Prozessoreinheit 27 ermöglicht die gerätetechnische Ablaufsteuerung und Auswertung der Messergebnisse.

Wie in Fig. 2 gezeigt, ist das Sammelelement 14 aus einem flachen Substrat 28, insbesondere Edelstahlblech durch Ätzen einstückig ausgeformt. Hierbei ist an dem Substratteil 28 ein distal abstehender, nadelförmiger Stechschaft 30 angeformt, während im Bereich einer zentralen Substratausnehmung 32 ein vorgebogener Transferteil 34 freigespart ist. Zwischen dem Stechschaft 30 und dem Transferteil 34 verläuft durchgehend ein rillenförmig halboffener Kapillarkanal 36 zum Transport der durch einen Einstich in ein Körperteil gewonnenen Blutprobe.

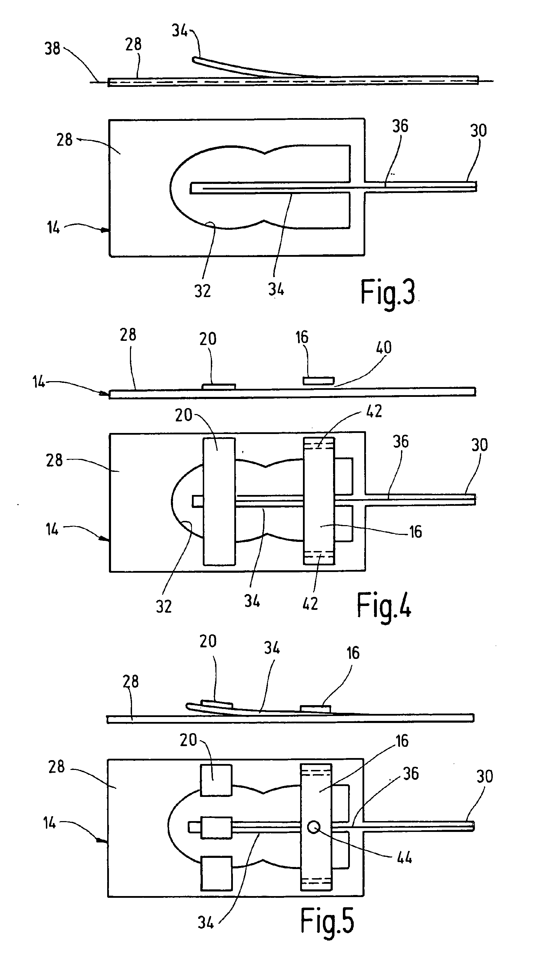

Wie auch aus Fig. 3 ersichtlich, ist der Transferteil 34 als gekrümmter Biegearm aus der Ebene 38 des Substrats 28 heraus abgebogen, und zwar nach derjenigen Seite hin, an welcher der Kanal 36 seitlich offen ist. Die Vorbiegung kann beispielsweise dadurch bewirkt sein, dass der Biegearm 34 unter Einwirkung von inneren Spannungen und/oder Oberflächenspannungen eine Biegeverformung erfährt. Speziell beim Ätzprozess können durch unterschiedliche Ätztiefen bzw. asyymetrisches Ätzen an den gegenüberliegenden Substratseiten des Transferteils 34 resultierende innere Biegespannungen bestehen bleiben. Eine weitere Möglichkeit besteht in einer einseitigen physikalischen oder chemischen Oberflächenveränderung bzw. Behandlung. Denkbar ist es auch, dass durch Auflaminieren einer weiteren Folienlage auf das Transferteil 34 ein zweischichtiger Streifen entsteht, der aufgrund unterschiedlicher Längenausdehnungskoeffizienten nach Art eines Bimetallstreifens unter Wärmeeinwirkung auslenkbar ist.

Gemäß Fig. 4 wird der Transferteil 34 durch einen lösbaren Niederhalter 20 in der Substratebene 38 gehalten. Der Niederhalter 20 kann beispielsweise ein Klebestreifen sein, der auf das Substrat 28 aufgeklebt ist und dabei die Ausnehmung 32 und den proximalen Abschnitt des Transferteils 34 so überspannt, dass die Abkrümmung des Transferteils 34 niedergehalten wird. Auf diese Weise wird im Ausgangszustand ein lichter Freiraum 40 zu dem Testelement 16 hin freigehalten, wodurch zunächst keine fluidische Verbindung zwischen dem Sammelkanal 36 und der zugewandten Seite des Testelements 16 besteht. Der Luftspalt 40 verhindert also, dass in dem Sammelkanal 36 gesammelte Blutflüssigkeit unmittelbar auf das Testelement 16 übertragen wird.

Das Testelement 16 kann durch einen Reagenzstreifen gebildet sein, der mit Trockenchemikalien beschichtet ist, die auf einen Analyten (Glucose) in einer Körperflüssigkeit (Blut, ggf. auch Gewebeflüssigkeit) beispielsweise durch Farbveränderung ansprechen, so dass ein optischer Nachweis möglich ist. Der Reagenzstreifen 16 wird durch Abstandshalter 42 im Bereich des Substrats 28 getragen, um den Freiraum 40 zu dem niedergehaltenen Transferteil 34 zu schaffen.

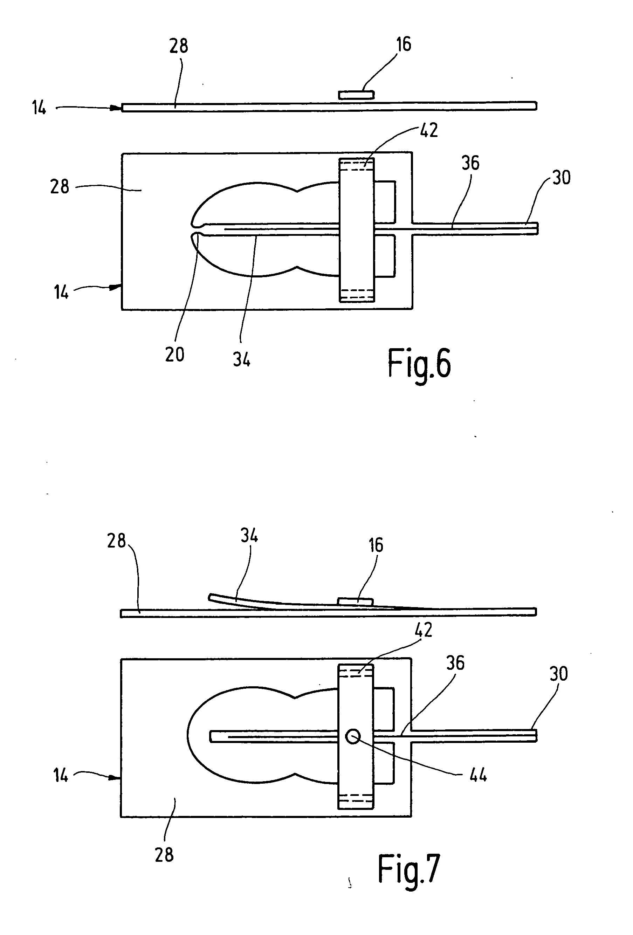

Wie in Fig. 5 veranschaulicht, kann der Niederhalter 20 mittels des Auslösers 22 gelöst bzw. aufgetrennt werden, so dass der Transferteil 34 aufgrund der eingeprägten Vorspannung bzw. Vorbiegung zu einem definierten Auslösezeitpunkt seitlich ausgelenkt wird und der Kanal 36 in fluidischen Kontakt mit dem Testelement 16 kommt. Dies kann dadurch bewerkstelligt werden, dass der Auslöser 22 einen Laser 46 (Fig. 1) als Trenneinheit ansteuert, um den Niederhalter 20 beidseitig von dem Transferteil 34 zu durchschneiden. Dabei krümmt sich der Transferteil 34 selbsttätig nach oben, so dass ein Kontaktzustand mit dem Testelement 16 erreicht und über die längsseitige Kanalöffnung Blutflüssigkeit als Probenfleck 44 übertragen wird. Aufgrund der Abkrümmung des Transferteils 34 kann der Eingriff unter Einhaltung eines vorgegebenen Kontaktwinkels erreicht werden, wodurch eine örtlich gezielte Blutübertragung mit einer hohen Positioniergenauigkeit möglich ist.

Anstelle einer berührungslosen Trenneinheit 46 kann auch ein mechanisches Schneidmittel vorgesehen sein, um den Niederhalter 20 zu durchtrennen. Möglich ist es auch, dass ein über das Transferteil 34 laminierter Niederhalter eine Doppelfunktion erfüllt, indem einerseits eine Rückhaltung und andererseits durch thermische Aktivierung bzw. Wärmeeinwirkung nach Art eines Bimetalls eine Auslenkung erzielt wird.

Bei der in Fig. 6 und 7 gezeigten Ausführungsform sind gleiche oder ähnliche Teile mit denselben Bezugszeichen wie vorstehen beschrieben versehen. Der Niederhalter ist hier durch eine trennbare Materialbrücke in Form eines Stegs 20 zwischen dem Substrat 28 und dem proximalen Ende des Transferteils 34 gebildet. Dieser Steg 20 kann bei der Herstellung durch nicht vollständiges Durchätzen des Substrats 28 erhalten werden, so dass keine zusätzlichen Bauteile bzw. Produktionsschritte erforderlich sind. Die Auslösung erfolgt dann nach Durchtrennen dieser verbleibenden Verbindung 20 unter Abkrümmung des Transferteils 34 in die eingeprägte Biegeform (Fig. 7).

Bei der Durchführung eines Tests wird eine Testeinheit 12 zweckmäßig aus einem Gerätemagazin aktiviert, um mittels des Stechantriebs 24 in einer hin und her gehenden Stechbewegung über einen Hauteinstich Körperflüssigkeit zu gewinnen. Der Flüssigkeitstransport in dem Kanal 36 erfolgt dann durch Kapillarwirkung, bis auch der Sammelbereich in dem Transferteil 34 hinreichend gefüllt ist. Von dort lässt sich durch die geräteseitige Auslösung die Probe 44 auf das Testelement 16 übertragen, wobei durch den definierten Triggerzeitpunkt konstante Messbedingungen bei jedem Test geschaffen werden. Nach der Auswertung mittels des Prozessors 27 wird das.Messergebnis dem Benutzer angezeigt, und die gebrauchte Testeinheit 12 wird als Wegwerfprodukt entsorgt.

## Patentansprüche

1. Testeinheit zum Untersuchen von Körperflüssigkeiten wie Blut, mit einem Sammelelement (14), das einen vorzugsweise als halboffener Kanal ausgebildeten mikrofluidischen Sammelbereich (36) für die Körperflüssigkeit aufweist, und einem Testelement (16), das durch Auslenkung eines Transferteils (34) des Sammelelements (14) mit dem Sammelbereich (36) zur Übertragung von Körperflüssigkeit in fluidische Verbindung bringbar ist, **dadurch gekennzeichnet, dass** der Transferteil (34) gegen einen Niederhalter (20) vorgespannt ist, wobei durch Lösen des Niederhalters (20) die Auslenkung des Transferteils (34) zu dem Testelement (16) hin auslösbar ist.

2. Testeinheit nach Anspruch 1, **dadurch gekennzeichnet, dass** der Sammelbereich (36) zumindest teilweise über das Transferteil (34) verläuft, wobei sich der Sammelbereich (36) und das Testelement (16) vor der Auslenkung körperlich getrennt im Abstand voneinander und nach der Auslenkung in Kontakt miteinander befinden.

3. Testeinheit nach Anspruch 1 oder 2, **dadurch gekennzeichnet, dass** der Transferteil (34) mit einer Vorbiegung vorzugsweise als Biegearm an dem Sammelelement (14) angebracht ist.

4. Testeinheit nach einem der Ansprüche 1 bis 3, **dadurch gekennzeichnet, dass** der Transferteil (34) durch Einwirkung innerer Spannungen und/oder Oberflächenspannungen unter Biegeverformung auslenkbar ist.

5. Testeinheit nach einem der Ansprüche 1 bis 4, **dadurch gekennzeichnet, dass** der Transferteil (34) durch eine unterschiedliche Oberflächenbehandlung an verschiedenen Seiten eine die Auslenkung bewirkende Biegespannung aufweist.

6. Testeinheit nach einem der Ansprüche 1 bis 4, **dadurch gekennzeichnet, dass** der Transferteil (34) durch unterschiedliche Materiallagen nach Art eines Bimetallstreifens auslenkbar ist.

7. Testeinheit nach einem der Ansprüche 1 bis 4, **dadurch gekennzeichnet, dass** der Transferteil (34) durch einen Ätzprozess aus einem flachen Substrat (28) gebildet ist, wobei durch unterschiedliche Ätztiefen an verschiedenen Seiten des Transferteils (34) resultierende innere Biegespannungen in dem Transferteil (34) bestehen.

8. Testeinheit nach einem der Ansprüche 1 bis 7, **dadurch gekennzeichnet, dass** das Sammelelement (14) ein Stechorgan (30) zum Einstechen in ein Körperteil aufweist, wobei der Sammelbereich (36) über den Einstich mit Körperflüssigkeit beaufschlagbar ist.

9. Testeinheit nach einem der Ansprüche 1 bis 8, **dadurch gekennzeichnet, dass** der Niederhalter durch eine trennbare Materialbrücke (20) zwischen dem Sammelelement (14) und dem Transferteil (34) gebildet ist, und dass die Auslenkung des Transferteils (34) unter Durchtrennen der Materialbrücke (20) auslösbar ist.

10. Testeinheit nach einem der Ansprüche 1 bis 9, **dadurch gekennzeichnet, dass** das Sammelelement (14) und der Niederhalter (20) aus einem Flachmaterial, insbesondere einem Edelstahlblech einstückig geformt sind, wobei der Niederhalter (20) durch mindestens einen trennbaren Steg gebildet ist.

11. Testeinheit nach einem der Ansprüche 1 bis 8, **dadurch gekennzeichnet, dass** der Niederhalter durch einen auf das Sammelelement (14) aufgebrachten und dabei einen Abschnitt des Transferteils (34) übergreifenden Klebestreifen (20) oder ein Laminat gebildet ist.

12. Testeinheit nach einem der Ansprüche 1 bis 11, **dadurch gekennzeichnet, dass** das Testelement (16) im Ausgangszustand vorzugsweise durch einen Abstandshalter (42) auf dem Sammelelement (14) in einem vorgegebenen Abstand zu dem Transferteil (34) angeordnet ist.

13. Testeinheit nach einem der Ansprüche 1 bis 12, **dadurch gekennzeichnet, dass** das Sammelelement (14) und der Transferteil (34) eine als disposibles Einmalprodukt bestimmte Baueinheit bilden.

14. Testeinheit nach einem der Ansprüche 1 bis 13, **dadurch gekennzeichnet, dass** der Transferteil (34) im Kontaktzustand unter Einhaltung eines vorgegebenen Kontaktwinkels mit dem Testelement (16) in Eingriff kommt.

15. Testeinheit nach einem der Ansprüche 1 bis 14, **dadurch gekennzeichnet, dass** das Testelement (16) ein über eine Öffnung des Sammelbereichs (36) mit der Körperflüssigkeit beaufschlagbares Reagenzfeld aufweist.

16. Testsystem zum Untersuchen von Körperflüssigkeiten wie Blut, mit
a) einem Sammelelement (14), das einen vorzugsweise als halboffener Kanal ausgebildeten mikrofluidischen Sammelbereich (36) für die Körperflüssigkeit aufweist,
b) einem Testelement (16), das durch Auslenkung eines Transferteils (34) des Sammelelements (14) mit dem Sammelbereich (36) zur Übertragung von Körperflüssigkeit in fluidische Verbindung bringbar ist, und
c) einem Gerät (10) zum Einsetzen mindestens eines Sammelelements (14), um einen Analyten in der Körperflüssigkeit zu erfassen,
**dadurch gekennzeichnet, dass**
d) der Transferteil (34) gegen einen Niederhalter (20) an dem Sammelelement (14) vorgespannt ist,
wobei durch Lösen des Niederhalters (20) mittels eines geräteseitigen Auslösers (22) die Auslenkung auslösbar ist.

17. Testsystem nach Anspruch 16, **dadurch gekennzeichnet, dass** der Auslöser (22) durch eine berührungslos arbeitende Trenneinheit, insbesondere einen Laser (46) gebildet ist.

18. Testsystem nach Anspruch 16, **dadurch gekennzeichnet, dass** der Auslöser (22) ein mechanisch wirksames Schneidmittel aufweist.

19. Testsystem nach Anspruch 16, **dadurch gekennzeichnet, dass** der Auslöser (22) durch einen thermischen Aktuator gebildet ist.

20. Testsystem nach einem der Ansprüche 15 bis 19, **dadurch gekennzeichnet, dass** das Testelement (16) unabhängig von dem Sammelelement (14) handhabbar ist.
